# EUROPEAN PATENT SPECIFICATION

(11) **EP 4 689 609 B1**
(45) Date of publication and mention of the grant of the patent: **12.08.2026**
(21) Application number: 24712846.5
(22) Date of filing: 25.03.2024
(51) Int. Cl.: G01N 21/31, G01N 33/04, A61B 5/00, A61B 5/145, A61B 5/1455, G01N 33/487, A61M 1/06

(54) **SYSTEM AND METHOD FOR DETERMINING CHARACTERISTICS OF A MILK-CONTAINING SYSTEM**
SYSTEM UND VERFAHREN ZUR BESTIMMUNG VON EIGENSCHAFTEN EINES MILCHHALTIGEN SYSTEMS
SYSTÈME ET PROCÉDÉ DE DÉTERMINATION DE CARACTÉRISTIQUES D'UN SYSTÈME CONTENANT DU LAIT

(30) Priority: 30.03.2023 EP 23165395
(43) Date of publication of application: 11.02.2026
(73) Proprietor: Koninklijke Philips N.V., 5656 AE Eindhoven (NL)
(72) Inventor: FERNANDO, Shakith Devinda, 5656 AG Eindhoven (NL); VAN BREE, Karl Catharina, 5656 AG Eindhoven (NL); LUCASSEN, Gerhardus Wilhelmus, 5656 AG Eindhoven (NL); PAULUSSEN, Elvira Johanna Maria, 5656 AG Eindhoven (NL)
(74) Representative: Philips Intellectual Property & Standards
(86) International application number: PCT/EP2024/057898
(87) International publication number: WO 2024/200328

(56) References cited:
- WO-A1-2018/087523
- RINALDO CUBEDDU ET AL: "Photonics for Life", IEEE PULSE, IEEE, USA, vol. 2, no. 3, 1 May 2011 (2011-05-01), pages 16 - 23, XP011326601, ISSN: 2154-2287, DOI: 10.1109/MPUL.2011.941519
- BOSSCHAART NIENKE ET AL: "Diffuse optical spectroscopic imaging of the human lactating breast", PROGRESS IN BIOMEDICAL OPTICS AND IMAGING, SPIE - INTERNATIONAL SOCIETY FOR OPTICAL ENGINEERING, BELLINGHAM, WA, US, vol. 11920, 9 December 2021 (2021-12-09), pages 119200N - 119200N, XP060150331, ISSN: 1605-7422, ISBN: 978-1-5106-0027-0, DOI: 10.1117/12.2615233

## Description

### FIELD OF THE INVENTION

The invention relates to the field of determining characteristics of a milk-containing system, and in particular to characteristics of a system containing human breast milk.

### BACKGROUND OF THE INVENTION

Human breast milk contains all the most important nutrients for human newborns and infants. However, only 40% of mothers exclusively breastfeed during the first six months from birth (see WHO and UNICEF, "Global Breastfeeding Scorecard, 2019: Increasing commitment to breastfeeding through funding and improved policies and programmes", WHO/NMH/NHD/19.22).

There are a number of reasons for the low rate of exclusive breastfeeding. Many of these reasons relate to experiencing difficulties in breastfeeding. In particular, many mothers have a perception that their milk supply is insufficient. Some mothers experience lactiferous duct blockage. Mothers who use breast pumps can find that the breast pump is not suitable for their milk ejection reflex.

There is therefore a need for improved aid for breastfeeding mothers.

WO2018/087523A1 provides an example of a handheld, integrated device for determining the scattering and/or absorption properties of a medium by performing optical time- of-flight measurements.

Cubeddu et al. ('Photonics for Life', IEEE Pulse, May/June 2011, pp. 16-23) describe how advances in photonics, particularly in optical technologies like lasers and fiber optics, enable non-invasive biomedical diagnostics and imaging in the field of biophotonics.

### SUMMARY OF THE INVENTION

The invention is defined by the claims.

According to examples in accordance with an aspect of the invention, there is provided a processing system for determining one or more characteristics of a milk-containing system containing human breast milk, the processing system being configured to: obtain, from at least one SPAD sensor, a distribution of photon arrival times at a plurality of pixels of the at least one SPAD sensor for photons emitted by a light source and reflected from within or transmitted through the milk-containing system; and process the distribution of photon arrival times to determine one or more characteristics of the milk-containing system, wherein the photons comprise first photons having a wavelength within a first wavelength range and second photons having a wavelength within a second, different wavelength range, wherein each of the first wavelength range and the second wavelength range is a different one of the following: a wavelength range coinciding with an absorption peak of water; a wavelength range coinciding with an absorption peak of lipids; and a wavelength range coinciding with an absorption peak of beta carotene.

The inventors have recognized that photon counting with time-domain spectroscopy can be used to provide information relating to a milk-containing system. In particular, the milk-containing system may contain human breast milk. For instance, the milk-containing system may be a lactating breast or a bottle of expressed breast milk.

This information may, for example, be provided to a breastfeeding mother, allowing the mother to make informed decisions regarding their breastfeeding, or used to generate breastfeeding guidance for the mother for an enhanced breastfeeding experience.

The one or more characteristics may, for example, include a lipid content of the milk, a beta carotene content of the milk, a water content of the milk and/or a riboflavin content of the milk. In the case where the milk-containing system comprises a lactating breast, the one or more characteristics may additionally or alternatively include one or more of a time of a milk ejection reflex event, a milk duct flow, a location of a milk duct blockage, a severity of a milk duct blockage and/or a change in milk supply over time.

In some examples, the milk-containing system is a breast of a human subject; and the processing system is configured to determine the one or more characteristics by: processing the distribution of photon arrival times to generate a time-varying milk duct dilation signal; and processing the time-varying milk duct dilation signal to determine the one or more characteristics.

The inventors have recognized that milk duct dilation during milk ejection is correlated with milk flow, and that a milk duct dilation signal can be generated using photon counting with time-domain spectroscopy.

This technique has numerous advantages over existing techniques for measuring milk duct dilation. In particular, the use of light provides a safer measurement than ultrasound, and a light source and SPAD sensor for obtaining the data relating to the distribution of photon arrival times may be provided at a lower cost than an ultrasound system. Compared with existing optical techniques (e.g. CMOS image sensing or OCT-Doppler flow imaging), photon counting using a SPAD sensor allows fast changes in milk duct diameter during milk ejection reflex events to be detected and provides three-dimensional depth information.

In some examples, the photons further comprise third photons having a wavelength within a third wavelength range, different to the first and second wavelength ranges, wherein each of the first wavelength range, the second wavelength range and the third wavelength range is a different one of the following: a wavelength range coinciding with an absorption peak of water; a wavelength range coinciding with an absorption peak of lipids; and a wavelength range coinciding with an absorption peak of beta carotene; and the processing system is configured to generate the milk duct generation signal by: for an initial time window: processing the distribution of photon arrival times to determine, for each pixel, a first absorption coefficient for each of the first, second and third wavelength ranges; processing the first absorption coefficients to determine, for each pixel, a concentration value for each of water, lipids and beta carotene; processing the concentration values for each pixel to identify pixels corresponding to a milk duct; and, for subsequent time windows: processing the data relating to the distribution of photon arrival times at each time window to determine a second absorption coefficient for one of the first, second and third wavelength ranges for at least one pixel of the identified pixels; processing each second absorption coefficient for each time window for the at least one pixel to generate the milk duct dilation signal.

This embodiment is based on the recognition that the concentration of milk is correlated with milk duct dilation. Preferably, a distribution of photon arrival times from a SPAD sensor positioned at a front of the nipple is used for this embodiment.

The inventors have further recognized that the concentration values can be used to distinguish between breast milk in a milk duct and breast tissue, since the concentrations of water, lipids and beta carotene are significantly different for the two. The concentration of milk is then generated based on concentration values for the milk duct.

In some examples, the photons further comprise fourth photons having a wavelength within a fourth wavelength range coinciding with an absorption peak of oxyhemoglobin and fifth photons having a wavelength within a fifth wavelength range coinciding with an absorption peak of deoxyhemoglobin; the second absorption coefficient is determined for the wavelength range coinciding with an absorption peak of lipids; and the processing system is further configured to: process the distribution of photon arrival times to determine an oxyhemoglobin and deoxyhemoglobin absorption coefficient; and generate the milk duct dilation signal by processing the second absorption coefficient and the oxyhemoglobin and deoxyhemoglobin absorption coefficient.

Since oxyhemoglobin and deoxyhemoglobin have a similar absorption coefficient to lipids, the accuracy of a milk duct dilation signal generated based on a lipids absorption coefficient can be improved by normalizing the lipids absorption coefficient using the oxyhemoglobin and deoxyhemoglobin absorption coefficient.

In some examples, the processing system is configured to generate the milk duct dilation signal by: processing the distribution of photon arrival times to determine a change in the distribution over time for each of the first and second wavelength ranges; and processing the change in the distribution over time for each of the first and second wavelength ranges to generate the milk duct dilation signal.

This embodiment is based on the recognition that the depth of the milk duct varies with dilation. Since the distribution of arrival times is correlated with tissue depth, changes in the distribution are correlated with changes in the diameter of the milk duct as the milk duct dilates. Preferably, a distribution of photon arrival times from a SPAD sensor positioned at a side of the nipple is used for this embodiment.

The change in the distribution over time for each of the first and second wavelength ranges may comprise a change in a peak of the distribution or a variation in a time-spread of the distribution.

In some examples, the processing system is further configured to filter the distribution of photon arrival times based on predetermined anatomical information.

The use of predetermined anatomical information allows the distribution to be filtered to remove at least some of the photons reflected from breast tissue.

The predetermined anatomical information may, for instance, include a typical anatomical depth of lipids and/or a typical anatomical depth of a milk duct.

In some examples, the processing system is configured to process the time-varying milk duct dilation signal by: receiving at least one historical time-varying milk duct dilation signal for the subject; and processing the time-varying milk duct dilation signal and the historical time-varying milk duct signal to determine the one or more characteristics.

This allows long-term changes in milk supply to be determined.

In some examples, the processing system is configured to: generate a plurality of milk duct dilation signals, each corresponding to a different subset of pixels; and process the plurality of milk duct dilation signals to determine the one or more characteristics.

In some examples, the at least one SPAD sensor comprises a plurality of SPAD sensors, each configured to obtain a distribution of photon arrival times for photons reflected from a different region of the breast; and the processing system is configured to: generate a plurality of milk duct dilation signals, each generated using the distribution of photon arrival times of a different SPAD sensor; and spatially correlate the plurality of milk duct dilation signals to identify a location of a milk duct blockage.

Early identification of milk duct blockages can reduce a likelihood of mastitis occurring.

In some examples, the one or more characteristics comprise a lipids content, a water content and/or a beta carotene content of milk in the milk-containing system; the photons further comprise third photons having a wavelength within a third wavelength range, different to the first and second wavelength ranges, wherein each of the first wavelength range, the second wavelength range and the third wavelength range is a different one of the following: a wavelength range coinciding with an absorption peak of water; a wavelength range coinciding with an absorption peak of lipids; and a wavelength range coinciding with an absorption peak of beta carotene; and the processing system is configured to determine the lipids content, water content and/or beta carotene content of milk in the milk-containing system by: processing the distribution of photon arrival times to determine an absorption coefficient for each of the first, second and third wavelength ranges; processing the absorption coefficients to determine a concentration value for each of water, lipids and beta carotene; and processing the concentration values to determine a lipids content, a water content and/or a beta carotene content of milk in the milk-containing system.

The lipids content and/or beta carotene content of milk can provide important information regarding the nutritional content of a mother's breast milk. The lipids content and/or beta carotene content may be determined from in vivo or in vitro embodiments.

In some examples, the processing system is further configured to process the one or more characteristics to generate breastfeeding guidance.

Where the milk-containing system comprises a lactating breast, the guidance may, for instance, relate to milk supply issues and/or milk duct blockages.

Breastfeeding guidance may also be provided based on the characteristics of expressed human breast milk. For instance, a recommendation to adjust a mother's diet may be generated based on the lipid or beta carotene content of the expressed milk.

There is also proposed a system for determining one or more characteristics of a milk-containing system containing human breast milk, the system comprising: a light source configured to emit photons towards the milk-containing system, wherein the light source is configured to emit photons comprising first photons having a wavelength within a first wavelength range and second photons having a wavelength within a second, different wavelength range, wherein each of the first wavelength range and the second wavelength range is a different one of the following: a wavelength range coinciding with an absorption peak of water; a wavelength range coinciding with an absorption peak of lipids; and a wavelength range coinciding with an absorption peak of beta carotene; at least one SPAD sensor, configured to measure an arrival time of photons reflected from within the milk-containing system; and the processing system described above.

There is also proposed a nipple-interface device, comprising: a cavity configured to receive a nipple; a light source configured to emit photons towards the cavity, wherein the light source is configured to emit photons comprising first photons having a wavelength within a first wavelength range and second photons having a wavelength within a second, different wavelength range, wherein each of the first wavelength range and the second wavelength range is a different one of the following: a wavelength range coinciding with an absorption peak of water; a wavelength range coinciding with an absorption peak of lipids; and a wavelength range coinciding with an absorption peak of beta carotene; and at least one SPAD sensor, configured to measure an arrival time of reflected photons.

A nipple-interface device may, for instance, be a breast pump (for determining the one or more characteristics during breast pumping), a nipple shield (for determining the one or more characteristics during breastfeeding), a lactation massage device or a breast milk collector.

According to another aspect of the invention, there is provided a method of determining one or more characteristics of a milk-containing system containing human breast milk, the method comprising: obtaining, from at least one SPAD sensor, a distribution of photon arrival times at a plurality of pixels of the at least one SPAD sensor for photons emitted by a light source and reflected from within the milk-containing system, wherein the photons comprise first photons having a wavelength within a first wavelength range and second photons having a wavelength within a second, different wavelength range, wherein each of the first wavelength range and the second wavelength range is a different one of the following: a wavelength range coinciding with an absorption peak of water; a wavelength range coinciding with an absorption peak of lipids; and a wavelength range coinciding with an absorption peak of beta carotene; and processing the distribution of photon arrival times to determine one or more characteristics of the milk-containing system.

There is also proposed a computer program product comprising computer program code means which, when executed on a computing device having a processing system, cause the processing system to perform all of the steps of the method described above.

These and all other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for determining one or more characteristics of a milk-containing system, according to an embodiment of the invention;
Fig. 2 illustrates a first method of generating a time-varying milk duct dilation signal;
Fig. 3 illustrates an example decision tree for classifying pixels as corresponding to a milk duct or breast tissue;
Fig. 4 illustrates a second method of generating a time-varying milk duct dilation signal;
Fig. 5 illustrates a computer-implemented method of determining one or more characteristics of a milk-containing system, according to an embodiment of the invention;
Fig. 6 illustrates a system for determining one or more characteristics of a milk-containing system, according to another embodiment of the invention;
Fig. 7 illustrates a system for determining one or more characteristics of a milk-containing system, according to another embodiment of the invention; and
Fig. 8 illustrates a system for determining one or more characteristics of a milk-containing system, according to another embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

There is proposed a system and method for determining one or more characteristics of a milk-containing system. A distribution of photon arrival times for at least two wavelengths is obtained from at least one SPAD sensor configured to detect photons arriving from within the milk-containing system. The at least two wavelengths correspond to absorption of two of water, lipids and beta carotene. The distribution of photon arrival times is processed to determine one or more characteristics of the milk-containing system.

Embodiments are at least partly based on the realization that photon counting with time-domain near-infrared spectroscopy can provide information about the composition of milk within a milk-containing system, and information about milk ducts within a human breast (e.g. milk ejection reflex events and milk flow).

Illustrative embodiments may, for example, be employed in breastfeeding systems and in breast milk expression systems.

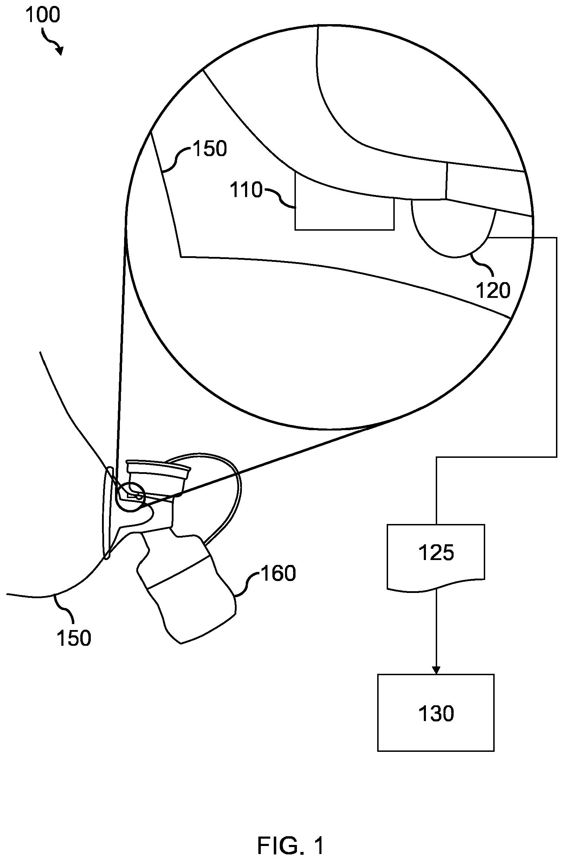

Fig. 1 illustrates a system 100 for determining one or more characteristics of a milk-containing system 150, according to an embodiment of the invention. The system 100 comprises a light source 110, at least one SPAD sensor 120, and a processing system 130. The processing system is, itself, an embodiment of the invention.

In Fig. 1, the milk-containing system 150 is a breast of a human subject. However, the system 100 is not limited to the determination of characteristics of a lactating breast, and the milk-containing system may be any system that contains milk, in particular human breast milk. For example, the milk-containing system may comprise a bottle of expressed breast milk.

The light source 110 is configured to emit photons towards the milk-containing system. The photons emitted by the light source comprise photons of at least two different wavelengths: a first wavelength within a first wavelength range, and a second, different wavelength within a second wavelength range. The first and second wavelength ranges are each a different one of: a wavelength range coinciding with an absorption peak of water; a wavelength range coinciding with an absorption peak of lipids; and a wavelength range coinciding with an absorption peak of beta carotene.

Suitable values for these wavelength ranges will be apparent to the skilled person. For instance, a wavelength range of 950-1000 nm may be used as a wavelength range coinciding with an absorption peak of water; a wavelength range of 900-950 nm may be used as the wavelength range coinciding with an absorption peak of lipids; and a wavelength range of 450-550 nm may be used as the wavelength range coinciding with an absorption peak of beta carotene.

When the milk-containing system 150 is a human breast, wavelengths in the ranges of 900-950 nm and 950-1000 nm will penetrate sufficiently deeply to provide information on most milk ducts (which have a depth distribution of around 1-8 mm). A wavelength in the range of 450-550 nm has a smaller penetration depth, so will only provide information on superficial milk ducts; however, this information may be sufficient for determining the one or more characteristics of the milk-containing system, especially given that the superficial milk ducts dilate and expand during milk ejection.

Suitable light sources for use as the light source 110 will be apparent to the skilled person. For instance, the light source may comprise a plurality of lasers, each laser configured to emit photons of a different wavelength.

The photons emitted by the light source are reflected (e.g. backscattered) from within or transmitted through the milk-containing system 150. The at least one SPAD sensor 120 is configured to measure an arrival time of these reflected/transmitted photons.

A SPAD (Single Photon Avalanche Diode) sensor is a type of image sensor that detects each individual photon incident on a pixel of the sensor. Each photon that enters a pixel is converted to an electric charge, and the resulting electrons are multiplied to form a large signal charge. The use of a SPAD sensor allows the time-of-flight of individual photons to be recorded; for instance, SPAD sensing has been used in brain imaging, with the direct time-of-flight computation using blood oxygenation as a marker (see Ban et al. (2022), "Kernel Flow: a high channel count scalable time-domain functional near-infrared spectroscopy system", J Biomed Opt, 27(7):074710).

In Fig. 1, the light source 110 and the at least one SPAD sensor 120 are integrated within a breast pump 160. However, the invention is not limited to this particular example, and the light source and at least one SPAD sensor may each be located in any suitable device.

For instance, where the milk-containing system is a human breast, the light source and at least one SPAD sensor may be contained within any suitable nipple-interface device comprising a cavity configured to receive a nipple. The light source may be configured to emit photons towards the cavity (i.e. towards a nipple received within the cavity). Examples of suitable nipple-interface devices include breast pumps, nipple shields, lactation massage devices and breast milk collectors (e.g. let-down collectors). A nipple interface device comprising a light source and at least one SPAD sensor as described herein is, itself, an embodiment of the invention.

The light source and at least one SPAD sensor may alternatively be contained in a dedicated milk-analysis device, or in separate devices. This may, in particular, be the case when the milk-containing system is outside the human body (e.g. where the milk-containing system is a bottle of expressed breast milk).

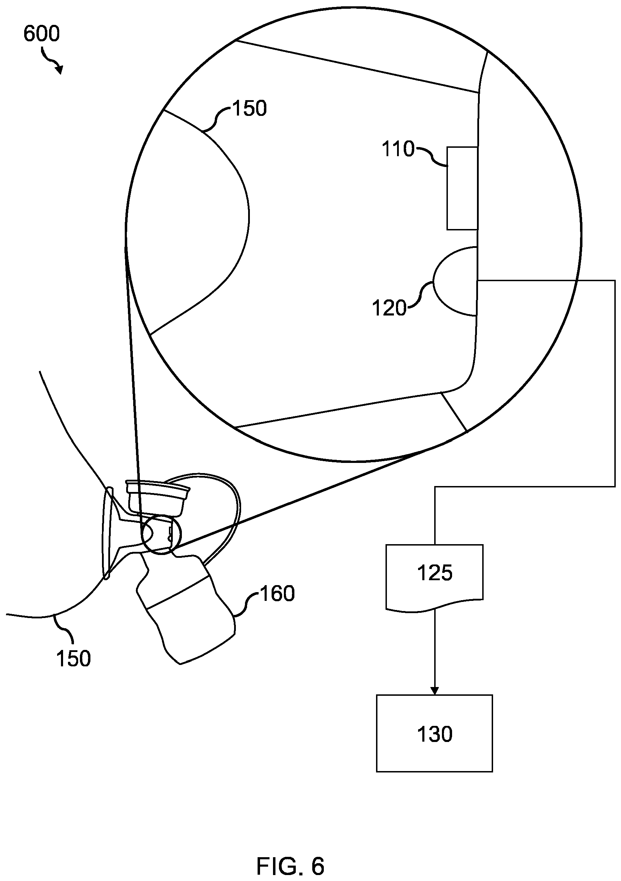

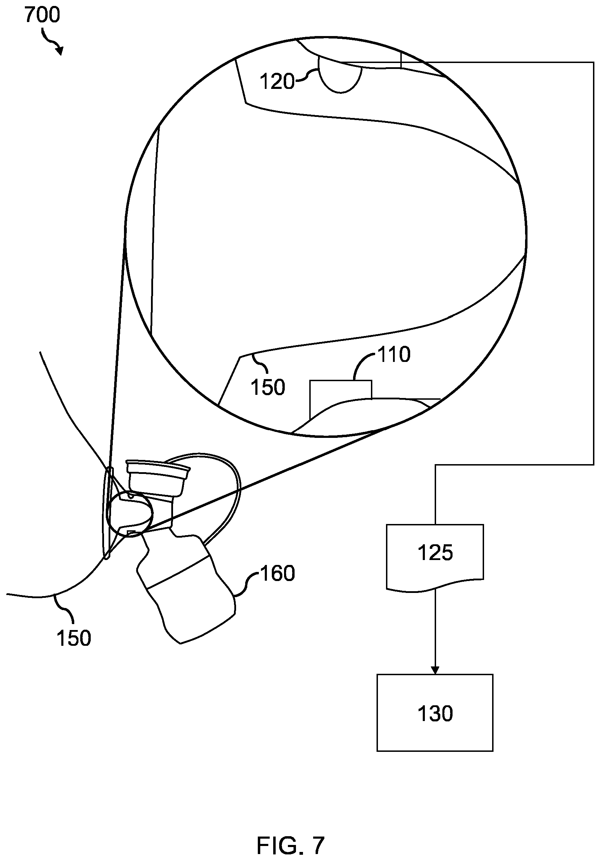

In Fig. 1, the light source 110 and the at least one SPAD sensor 120 are positioned at a side of the nipple (i.e. such that the direction of travel of the photons is substantially perpendicular to a direction of milk flow). The light source and the at least one SPAD sensor are positioned at a same side of the nipple, such that the at least one SPAD sensor detects photons emitted by the light source and reflected from within the milk-containing system. In other examples, the light source 110 and the at least one SPAD sensor 120 may be positioned at a front of the nipple (i.e. such that the direction of travel of the photons is substantially parallel to a direction of milk flow)(see Fig. 6), or the and light source and SPAD sensor can be arranged in an rectilinear set-up, where light source is on one side of the nipple area and the SPAD sensor on an opposite side (see Fig. 7).

Preferably, the light source 110 is configured to emit photons from a fixed position with respect to the milk-containing system. For instance, where the light source and the at least one SPAD sensor are provided in a breast pump, the emission of photons from the light source may be synchronized to the pumping frequency of the breast pump.

The processing system 130 is configured to obtain, from the at least one SPAD sensor 120, a distribution 125 of photon arrival times at a plurality of pixels of the at least one SPAD sensor for photons emitted by the light source and reflected or scattered from within the milk-containing system.

Having received the distribution 125 of photon arrival times, the processing system 130 is configured to process the distribution of photon arrival times to determine one or more characteristics of the milk containing system 150.

The one or more characteristics of the milk-containing system may comprise any characteristics that can be determined based on the distribution of photon arrival times from the milk-containing system 150. For instance, the one or more characteristics may comprise nutritional information relating to the milk within the milk-containing system, such as a lipid content, a beta carotene content, a water content and/or a riboflavin content (e.g. a percentage content). If the milk-containing system is a human breast, the one or more characteristics may additionally or alternatively comprise one or more breastfeeding characteristics, such as a time of a milk ejection reflex event, a milk duct flow, a location of a milk duct blockage, a severity of a milk duct blockage, and/or a change in milk supply over time.

In some examples, the one or more characteristics may comprise nutritional information relating to the composition of milk within the milk-containing system 150. These characteristics may be determined for any type of milk-containing system (i.e. for both in vivo and in vitro systems).

In order to determine this nutritional information, at least three different wavelengths are required, the wavelengths corresponding to water, lipids and beta carotene respectively. In other words, the photons emitted by the light source 110 further comprise photons having a wavelength within a third wavelength range, different to the first and second wavelength ranges, such that the photons emitted by the light source comprise photons having a wavelength within a wavelength range coinciding with an absorption peak of water, photons having a wavelength within a wavelength range coinciding with an absorption peak of lipids, and photons having a wavelength within a wavelength range coinciding with an absorption peak of beta carotene.

The processing system 130 is configured to determine a lipids content, a beta carotene content, and/or a water content of milk in the milk-containing system by processing the distribution 125 of photon arrival times to determine an absorption coefficient for each of the first, second and third wavelength ranges (i.e. absorption coefficients for water, lipids and beta carotene), and processing the absorption coefficients to determine a concentration value for each of water, lipids and beta carotene.

The absorption coefficients for water, lipids and beta carotene may, for instance, be determined by processing the distribution 125 of photon arrival times to determine the optical properties of the substance which reflected or scattered the photons using the method described in Liebert et al. (2003), "Evaluation of optical properties of highly scattering media by moments of distributions of times of flight of photons", Applied Optics, 42(28):5785-5792.

The concentration values for water, lipids and beta carotene may be determined from the absorption coefficients by applying a modification of the Beer-Lambert law, as expressed by the equation: $c = \frac{{μ}_{10} \left(λ\right)}{ε \left(λ\right)}$ where c is the concentration, *µ*₁₀(*λ*) is the absorption coefficient at wavelength *λ*, and *ε*(λ) is the expected molar absorption coefficient. The skilled person will be readily capable of determining an expected molar absorption coefficient for each of water, lipids and beta carotene.

The concentration values for water, lipids and beta carotene are processed for each wavelength to determine a water content, a lipids content and/or a beta carotene content.

In some examples, the distribution 125 of photon arrival times further comprises photons having at least one additional wavelength within a wavelength range coinciding with an absorption peak of riboflavin (e.g. a wavelength range of 500-800 nm), and the processing system 130 is further configured to determine a riboflavin content of milk in the milk-containing system. The processing system may determine the riboflavin content using the method described above with respect to water, lipids and beta carotene.

In some examples in which the milk-containing system is a human breast, the one or more characteristics may additionally or alternatively comprise one or more breastfeeding characteristics, such as a time of a milk ejection reflex event, a milk duct flow, a location of a milk duct blockage, a severity of a milk duct blockage, and/or a change in milk supply over time. The processing system 130 may be configured to determine these breastfeeding characteristics by processing the distribution 125 of photon arrival times to generate a time-varying milk duct dilation signal, and processing the time-varying milk duct dilation signal to determine the one or more characteristics.

A time-varying milk duct dilation signal is a signal responsive to changes in milk duct diameter. Various methods are proposed for generating a time-varying milk duct dilation signal based on the distribution 125 of photon arrival times.

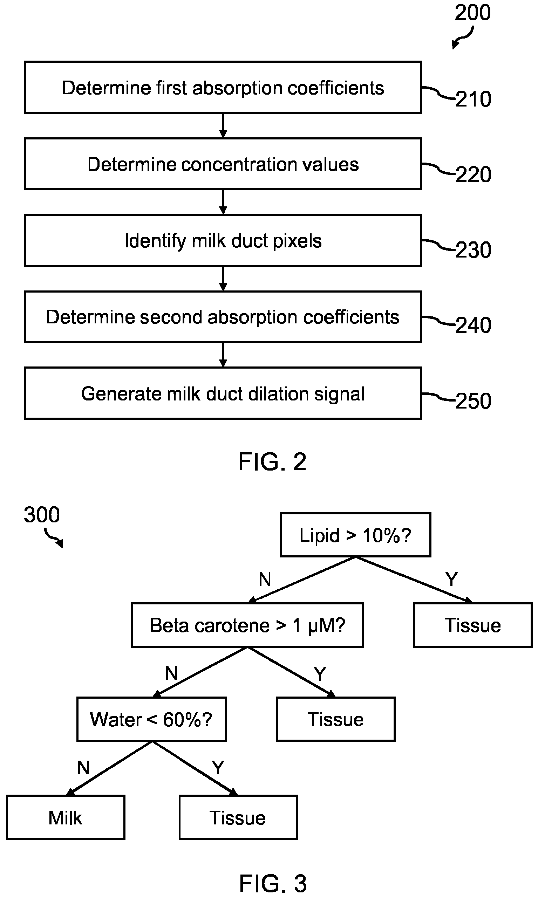

Fig. 2 illustrates a first method 200 of generating a time-varying milk duct dilation signal. This first method of generating a time-varying milk duct dilation is preferred when the SPAD sensor is positioned in front of the nipple.

At least three different wavelengths (corresponding to water, lipids and beta carotene respectively) are required to generate a time-varying milk duct dilation signal using this first method. In other words, the distribution 125 of photon arrival times used in this first method comprises arrival times for photons having a wavelength within a wavelength range coinciding with an absorption peak of water, photons having a wavelength within a wavelength range coinciding with an absorption peak of lipids, and photons having a wavelength within a wavelength range coinciding with an absorption peak of beta carotene.

The method 200 begins at step 210, in which the distribution of photon arrival times is processed, for an initial time window (e.g. a first sampling window), to determine, for each pixel of the at least one SPAD sensor, a first absorption coefficient for each of the three wavelength ranges.

In other words, an absorption coefficient for water is determined based on the arrival times of photons having a wavelength within the wavelength range coinciding with an absorption peak of water; an absorption coefficient for lipids is determined based on the arrival times of photons having a wavelength within the wavelength range coinciding with an absorption peak of lipids; and an absorption coefficient for beta carotene is determined based on the arrival times of photons having a wavelength within the wavelength range coinciding with an absorption peak of beta carotene.

At step 220, the first absorption coefficients are processed to determine, for each pixel of the SPAD sensor, a concentration value for each of water, lipids and beta carotene, for example, using the method described above.

At step 230, the concentration values for each pixel are processed to identify pixels corresponding to a milk duct (i.e. pixels at which the photons arrived from within the milk duct). The concentration values for water, lipids and beta carotene for each pixel may be compared with threshold or reference values to identify pixels corresponding to a milk duct.

The inventors have recognized that the concentration values for each pixel can be used to distinguish between milk (in milk ducts) and breast tissue, since milk and breast tissue have different relative amounts of water, lipids and beta carotene.

Known concentration values for lipids, water and beta carotene in breast tissue and milk ducts may be used to determine suitable threshold or reference values for determining whether each pixel corresponds to a milk duct or breast tissue (see, for example Nachabé et al. (2011), "Diagnosis of breast cancer using diffuse optical spectroscopy from 500 to 1600 nm: comparison of classification methods", J Biomed Opt 16(8):087010; Bosschaart et al. (2019), "Diffuse optical spectroscopic imaging for the investigation of human lactation physiology: a case study on mammary involution", J Biomed Opt 24(5):1-8; and Xavier et al. (2019), "In Vitro Digestion of Human Milk: Influence of the Lactation Stage on the Micellar Carotenoids Content", Antioxidants 8(8):291).

Fig. 3 illustrates an example decision tree 300 for classifying pixels as corresponding to a milk duct or breast tissue. Using decision tree 300, a lipids content of more than 10%, a beta carotene content of more than 1 µM, and/or a water content of less than 60% indicates breast tissue. Pixels for which the lipids content is less than 10%, the beta carotene content is less than 1 µM, and the water content is more than 60% are identified as pixels corresponding to milk ducts. The lipids content and water content may be determined as a percentage from the concentration values as described above.

As the skilled person will appreciate, the decision tree 300 of Fig. 3 illustrates only one set of suitable values for distinguishing between milk and breast tissue, and other, similar values may be used. For instance, suitable values for a threshold to distinguish between breast tissue and milk ducts may be in the range 10-80% for lipids, 10-12% for beta carotene and 30-60% for water.

Returning to Fig. 2, at step 240, the distribution 125 of photon arrival times is processed, at each subsequent time window, to determine a second absorption coefficient for at least one of water, lipids and/or beta carotene for at least one pixel of the identified milk duct pixels. The second absorption coefficient may be determined using the same technique used to determine the first absorption coefficients.

At step 250, each second absorption coefficient for each time window is processed for the at least one pixel to generate the time-varying milk duct dilation signal. The time-varying milk duct dilation signal is, in this case, a signal responsive to changes in a concentration of water, lipids and/or beta carotene in the at least one milk duct pixel. The concentration of water, lipids and/or beta carotene may be determined from the second absorption coefficient using the method described above.

In other words, having identified the pixels that correspond to milk ducts, the concentration of any one of water, lipids and beta carotene within the milk ducts is sufficient to determine a relative amount of milk within the milk ducts, allowing a signal representative of changes in the amount of milk (i.e. the milk duct dilation signal) to be generated.

As previously mentioned, the second absorption coefficient may be determined for any one of lipids, water and beta carotene. If the second absorption coefficient is an absorption coefficient for lipids (i.e. the second absorption coefficient is determined for the wavelength within the wavelength range coinciding with an absorption peak of lipids), additional wavelengths may be used to remove the time-varying effects of oxyhemoglobin and deoxyhemoglobin from the time-varying milk duct signal.

For instance, the photons emitted by the light source 110 may further comprise photons having a wavelength within a fourth wavelength range coinciding with an absorption peak of oxyhemoglobin and photons having a wavelength within a fifth wavelength range coinciding with an absorption peak of deoxyhemoglobin. The processing system 130 may process the distribution 125 of photon arrival times to determine an absorption coefficient for each of oxyhemoglobin and deoxyhemoglobin for the at least one milk duct pixel, and generate the time-varying milk duct dilation signal by processing the absorption coefficients for lipids, oxyhemoglobin and deoxyhemoglobin for the at least one milk duct pixel to normalize the concentration value for lipids. For instance, the time-varying milk duct dilation signal (when based on the absorption coefficient for lipids) may be divided by a time-varying signal responsive to the absorption coefficients for oxyhemoglobin and by a time-varying signal responsive to the absorption coefficients for deoxyhemoglobin in order to normalize the time-varying signal.

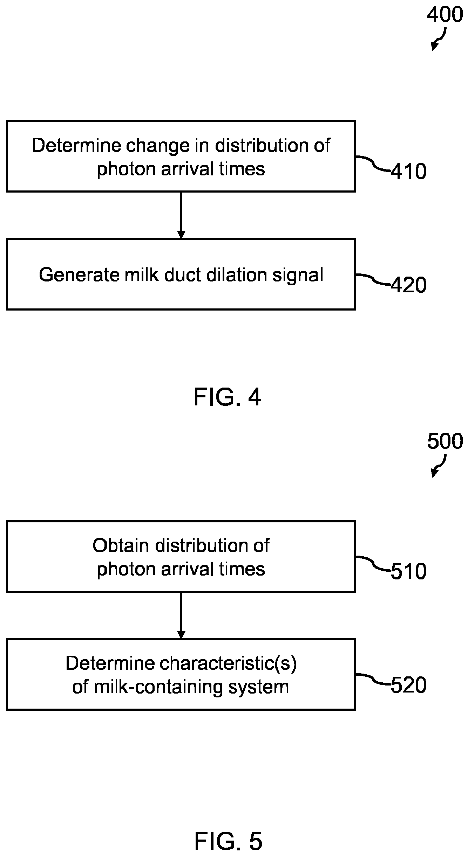

Fig. 4 illustrates a second method 400 of generating a time-varying milk duct dilation signal. This second method of generating a time-varying milk duct dilation is preferred when the SPAD sensor is positioned at a side of the nipple. A minimum of two different wavelengths (i.e. a wavelength within the first wavelength range and a wavelength within the second wavelength range) are required for this second method.

This second method of generating the time-varying milk duct dilation signal is based on the recognition that the distribution of photon arrival times changes as the diameter of the milk duct changes (due to dilation).

The second method 400 begins at step 410, at which the distribution 125 of photon arrival times is processing to determine a change in the distribution over time for each of the first and second wavelength ranges.

Any suitable measure may be used to determine a change in the distribution 125 over time. For instance, the change in the distribution over time may comprise a change in a peak of the distribution or a variation in a time-spread of the distribution. Other suitable measures of the change in the distribution over time will be apparent to the skilled person.

At step 420, the change in the distribution over time for each of the first and second wavelength ranges is processed to generate the time-varying milk duct dilation signal. In other words, the time-varying milk duct dilation signal is a signal responsive to changes in the distribution photon arrival times.

In some examples, the processing system 130 is configured to filter the distribution 125 of photon arrival times prior to generating the time-varying milk duct dilation signal. In other words, the time-varying milk duct dilation signal may, in some examples, be generated based on a filtered distribution 125 of photon arrival times, rather than on the raw distribution.

The distribution 125 of photon arrival times may be filtered based on predetermined anatomical information, to remove the arrival times of photons arriving from depths indicative of breast tissue. The predetermined anatomical information may be determined from population data.

For instance, the predetermined anatomical information may include an expected depth of adipose tissue, and a peak in the distribution of photon arrival times for the wavelength corresponding to lipids absorption at this depth may be used to filter out adipose tissue. In another example, the predetermined anatomical information may include an expected depth of a milk duct, and a peak in the distribution of photon arrival times for the wavelength corresponding to water absorption at this depth may be used to confirm a depth of the milk duct. The distribution may then be filtered to remove photons from other depths.

As stated above, the time-varying milk duct dilation signal may be processed to determine one or more characteristics of the milk-containing system. For instance, a time of a milk ejection reflex event may be determined by detecting a peak in the time-varying milk duct dilation signal. Milk flow information may be determined by detecting changes in the time-varying milk duct dilation signal, which correspond to changes in milk flow.

In some examples, the one or more characteristics may comprise a change in milk supply over time (e.g. a quantification of a long-term natural reduction in milk supply over a period of several months). The processing system 130 may be configured to determine a change in milk supply over time by receiving at least one historical time-varying milk duct dilation signal for the subject, and processing the time-varying milk duct dilation signal and the at least one historical time-varying milk duct dilation signal to determine the change to milk supply over time.

In some examples, the one or more characteristics may comprise a location of a milk duct blockage and/or determine a severity of a milk duct blockage. In order to identify a location of a milk duct blockage and/or determine a severity of a milk duct blockage, a plurality of milk duct dilation signals may be generated, each corresponding to milk duct dilation for a different region.

In some examples, the processing system 130 may be configured to process the distribution 125 of photon arrival times to generate a plurality of milk duct dilation signals, each signal corresponding to a different subset of pixels of the SPAD sensor. The processing system may process the plurality of milk duct dilation signals to determine the one or more characteristics.

In some examples, the at least one SPAD sensor 120 may comprise a plurality of SPAD sensors, each configured to obtain a distribution of photon arrival times for photons reflected from a different region of the breast. For instance, a plurality of SPAD sensors and light sources may be provided in a circular arrangement around the milk-containing system 150. The processing system 130 may be configured to generate a plurality of milk duct dilation signals, each generated using the distribution of photon arrival times of a different SPAD sensor, using any of the methods described above for generating a milk duct dilation signal.

The processing system 130 may spatially correlate the plurality of milk duct dilation signals to identify a location of a milk duct blockage and/or a determine a severity of a milk duct blockage. Suitable methods of spatially correlating the plurality of milk duct dilation signals will be apparent to the skilled person. A lack of correlation in milk duct dilation signals from neighboring regions is indicative of a milk duct blockage. The extent of the lack of correlation is indicative of a severity of a milk duct blockage.

Having determined the one or more characteristics of the milk-containing system using any of the methods described above, the processing system 130 may process the one or more characteristics to generate breastfeeding guidance. The type of breastfeeding guidance generated may depend on the one or more characteristics determined.

For instance, if the one or more characteristics include nutritional information (e.g. a lipids, water and/or beta carotene content of the milk), the breastfeeding guidance may comprise dietary recommendation. If the one or more characteristics include breastfeeding characteristics (e.g. a time of a milk ejection reflex, a milk flow, a location and/or severity of a milk duct blockage, and/or a change in milk supply over time), the breastfeeding guidance may comprise information about milk supply issues and/or milk duct blockages. Further examples of suitable breastfeeding guidance that may be generated based on one or more characteristics of a milk-containing system will be apparent to the skilled person.

In some examples, the generated breastfeeding guidance may be presented to a user (e.g. the mother whose milk is contained in the milk-containing system) by providing a user-perceptible output to an output user interface (not shown in Fig. 1) connected to or forming part of the system 100. For instance, the breastfeeding guidance may be presented to the user in the form of a textual display, an image (e.g. a visualization of a milk duct with an identification of a location of a blockage or a visualization of changes in milk duct diameter over time) and/or a sound. Further examples of suitable user-perceptible outputs will be apparent to the skilled person.

Fig. 5 illustrates a computer-implemented method 500 of determining one or more characteristics of a milk-containing system, according to an embodiment of the invention.

The method 500 begins at step 510, at which a distribution of photon arrival times at a plurality of pixels of at least one SPAD sensor is obtained from the at least one SPAD sensor. The distribution of photon arrival times comprises arrival times of photons emitted by a light source and reflected from within or transmitted through the milk-containing system. The photons comprise first photons having a wavelength within a first wavelength range and second photons having a wavelength within a second, different wavelength range. Each of the first wavelength range and the second wavelength range is a different one of: a wavelength range coinciding with an absorption peak of water, a wavelength range coinciding with an absorption peak of lipids, and a wavelength range coinciding with an absorption peak of beta carotene.

At step 520, the distribution of photon arrival times is processed to determine one or more characteristics of the milk-containing system.

Fig. 6 illustrates a system 600 for determining one or more characteristics of a milk-containing system, according to another embodiment of the invention. The system 600 is identical to the system 100, except that the light source 110 and the at least one SPAD sensor 120 are positioned at a front of the nipple, such that the direction of travel of the photons is substantially parallel to a direction of milk flow. The system 600 is particularly advantageous when using the first method 200 (described with respect to Fig. 2) to generate a time-varying milk duct dilation signal, and in particular for obtaining a distribution of photon arrival times for a wavelength within a wavelength range coinciding with an absorption peak of beta carotene, as wavelengths within this wavelength range do not penetrate very deeply into the tissue.

Fig. 7 illustrates a system 700 for determining one or more characteristics of a milk-containing system, according to another embodiment of the invention. The system 700 is identical to the system 100, except that the light source 110 and the at least one SPAD sensor 120 are arranged in a rectilinear set-up, with the light source and the at least one SPAD sensor are provided on opposite sides of the nipple. With this arrangement, the at least one SPAD sensor detects photons emitted by the light source and transmitted through (and scattered by) the milk-containing system 150.

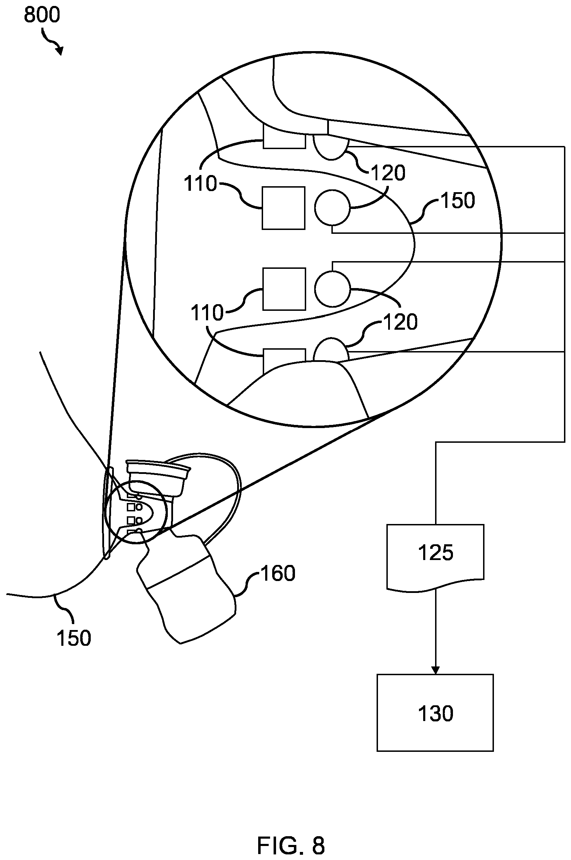

Fig. 8 illustrates a system 800 for determining one or more characteristics of a milk-containing system, according to another embodiment of the invention. The system 800 is identical to the system 100, except that the light source 110 and the at least one SPAD sensor 120 comprise a plurality of light sources 110 and SPAD sensors 120 provided in a circular arrangement around the nipple.

It will be understood that the disclosed methods are computer-implemented methods. As such, there is also proposed a concept of a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processing system may be implemented by a single processing system or by multiple separate processing units which may together be considered to constitute a "processor". Such processing units may in some cases be remote from each other and communicate with each other in a wired or wireless manner.

Ordinal numbers (e.g. "first", "second" and so on) have been used purely to distinguish different elements from one another for the sake of clarity, and reference to a non-"first" (e.g. "second" or "third") element does not necessitate that a "first" element be present. The skilled person would be capable of relabeling any such elements as appropriate (e.g. relabeling a "second" element as a "first" element if only the second element is present).

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A processing system (130) for determining one or more characteristics of a milk-containing system (150) containing human breast milk, the processing system being configured to:
process a distribution of photon arrival times to determine one or more characteristics of the milk-containing system,
**characterized in that** the processing system is configured to:
obtain, from at least one SPAD sensor (120), the distribution (125) of photon arrival times at a plurality of pixels of the at least one SPAD sensor for photons emitted by a light source (110) and reflected from within or transmitted through the milk-containing system, wherein
the photons comprise first photons having a wavelength within a first wavelength range and second photons having a wavelength within a second, different wavelength range, wherein each of the first wavelength range and the second wavelength range is a different one of the following:
a wavelength range coinciding with an absorption peak of water;
a wavelength range coinciding with an absorption peak of lipids; and
a wavelength range coinciding with an absorption peak of beta carotene.

2. The processing system (130) of claim 1, wherein:
the milk-containing system (150) is a breast of a human subject; and
the processing system is configured to determine the one or more characteristics by:
processing the distribution (125) of photon arrival times to generate a time-varying milk duct dilation signal; and
processing the time-varying milk duct dilation signal to determine the one or more characteristics.

3. The processing system (130) of claim 2, wherein:
the photons further comprise third photons having a wavelength within a third wavelength range, different to the first and second wavelength ranges, wherein each of the first wavelength range, the second wavelength range and the third wavelength range is a different one of the following:
a wavelength range coinciding with an absorption peak of water;
a wavelength range coinciding with an absorption peak of lipids; and
a wavelength range coinciding with an absorption peak of beta carotene; and
the processing system is configured to generate the milk duct generation signal by:
for an initial time window:
processing the distribution (125) of photon arrival times to determine, for each pixel, a first absorption coefficient for each of the first, second and third wavelength ranges;
processing the first absorption coefficients to determine, for each pixel, a concentration value for each of water, lipids and beta carotene;
processing the concentration values for each pixel to identify pixels corresponding to a milk duct;
and, for subsequent time windows:
processing the data relating to the distribution of photon arrival times at each time window to determine a second absorption coefficient for one of the first, second and third wavelength ranges for at least one pixel of the identified pixels;
processing each second absorption coefficient for each time window for the at least one pixel to generate the milk duct dilation signal.

4. The processing system (130) of claim 3, wherein:
the photons further comprise fourth photons having a wavelength within a fourth wavelength range coinciding with an absorption peak of oxyhemoglobin and fifth photons having a wavelength within a fifth wavelength range coinciding with an absorption peak of deoxyhemoglobin;
the second absorption coefficient is determined for the wavelength range coinciding with an absorption peak of lipids;
and the processing system is further configured to:
process the distribution (125) of photon arrival times to determine an oxyhemoglobin and deoxyhemoglobin absorption coefficient; and
generate the milk duct dilation signal by processing the second absorption coefficient and the oxyhemoglobin and deoxyhemoglobin absorption coefficient.

5. The processing system (130) of claim 2, wherein the processing system is configured to generate the milk duct dilation signal by:
processing the distribution (125) of photon arrival times to determine a change in the distribution over time for each of the first and second wavelength ranges; and
processing the change in the distribution over time for each of the first and second wavelength ranges to generate the milk duct dilation signal.

6. The processing system (130) of any of claims 2 to 5, wherein the processing system is further configured to filter the distribution (125) of photon arrival times based on predetermined anatomical information.

7. The processing system (130) of any of claims 2 to 6, wherein the processing system is configured to process the time-varying milk duct dilation signal by:
receiving at least one historical time-varying milk duct dilation signal for the subject; and
processing the time-varying milk duct dilation signal and the historical time-varying milk duct signal to determine the one or more characteristics.

8. The processing system (130) of any of claims 2 to 7, wherein the processing system is configured to:
generate a plurality of milk duct dilation signals, each corresponding to a different subset of pixels; and
process the plurality of milk duct dilation signals to determine the one or more characteristics.

9. The processing system (130) of any of claims 2 to 8, wherein:
the at least one SPAD sensor (120) comprises a plurality of SPAD sensors, each configured to obtain a distribution (125) of photon arrival times for photons reflected from a different region of the breast; and
the processing system is configured to:
generate a plurality of milk duct dilation signals, each generated using the distribution of photon arrival times of a different SPAD sensor; and
spatially correlate the plurality of milk duct dilation signals to identify a location of a milk duct blockage.

10. The processing system (130) of any of claims 1 to 9, wherein:
the one or more characteristics comprise a lipids content, a water content and/or a beta carotene content of milk in the milk-containing system;
the photons further comprise third photons having a wavelength within a third wavelength range, different to the first and second wavelength ranges, wherein each of the first wavelength range, the second wavelength range and the third wavelength range is a different one of the following:
a wavelength range coinciding with an absorption peak of water;
a wavelength range coinciding with an absorption peak of lipids; and
a wavelength range coinciding with an absorption peak of beta carotene; and
the processing system is configured to determine the lipids content, water content and/or beta carotene content of milk in the milk-containing system by:
processing the distribution (125) of photon arrival times to determine an absorption coefficient for each of the first, second and third wavelength ranges;
processing the absorption coefficients to determine a concentration value for each of water, lipids and beta carotene; and
processing the concentration values to determine a lipids content, a water content and/or a beta carotene content of milk in the milk-containing system.

11. The processing system (130) of any of claims 1 to 10, wherein the processing system is further configured to process the one or more characteristics to generate breastfeeding guidance.

12. A system (100, 600, 700, 800) for determining one or more characteristics of a milk-containing system (150) containing human breast milk, the system comprising:
a light source (110) configured to emit photons towards the milk-containing system, wherein the light source is configured to emit photons comprising first photons having a wavelength within a first wavelength range and second photons having a wavelength within a second, different wavelength range, **characterized in that** each of the first wavelength range and the second wavelength range is a different one of the following:
a wavelength range coinciding with an absorption peak of water;
a wavelength range coinciding with an absorption peak of lipids; and
a wavelength range coinciding with an absorption peak of beta carotene;
at least one SPAD sensor (120), configured to measure an arrival time of photons reflected from within the milk-containing system; and
the processing system (130) of any of claims 1 to 11.

13. A nipple-interface device (160), comprising:
a cavity configured to receive a nipple;
a light source (110) configured to emit photons towards the cavity, wherein the light source is configured to emit photons comprising first photons having a wavelength within a first wavelength range and second photons having a wavelength within a second, different wavelength range, wherein each of the first wavelength range and the second wavelength range is a different one of the following:
a wavelength range coinciding with an absorption peak of water;
a wavelength range coinciding with an absorption peak of lipids; and
a wavelength range coinciding with an absorption peak of beta carotene; and
at least one SPAD sensor (120), configured to measure an arrival time of reflected photons.

14. A method (500) of determining one or more characteristics of a milk-containing system (150) containing human breast milk, **characterized in that** the method comprises:
obtaining, from at least one SPAD sensor (120), a distribution (125) of photon arrival times at a plurality of pixels of the at least one SPAD sensor for photons emitted by a light source (110) and reflected from within or transmitted through the milk-containing system, wherein the photons comprise first photons having a wavelength within a first wavelength range and second photons having a wavelength within a second, different wavelength range, each of the first wavelength range and the second wavelength range is a different one of the following:
a wavelength range coinciding with an absorption peak of water;
a wavelength range coinciding with an absorption peak of lipids; and
a wavelength range coinciding with an absorption peak of beta carotene; and
processing the distribution of photon arrival times to determine one or more characteristics of the milk-containing system.

15. A computer program product comprising computer program code means which, when executed on a processing system (130) according to any of claims 1 to 11, cause the processing system (130) to perform all of the steps of the method (500) according to claim 14.

## Patentansprüche

1. Verarbeitungssystem (130) zur Bestimmung einer oder mehrerer Eigenschaften eines milchhaltigen Systems (150), das menschliche Muttermilch enthält, wobei das Verarbeitungssystem dazu ausgebildet ist:
eine Verteilung von Photonenankunftszeiten zu verarbeiten, um eine oder mehrere Eigenschaften des milchhaltigen Systems zu bestimmen,
**dadurch gekennzeichnet, dass** das Verarbeitungssystem weiter dazu ausgebildet ist:
von mindestens einem SPAD-Sensor (120) die Verteilung (125) von Photonenankunftszeiten an einer Vielzahl von Pixeln des mindestens einen SPAD-Sensors für Photonen zu erhalten, die von einer Lichtquelle (110) emittiert und von innerhalb des milchhaltigen Systems reflektiert oder durch dieses hindurch übertragen werden, wobei
die Photonen erste Photonen, die eine Wellenlänge innerhalb eines ersten Wellenlängenbereichs aufweisen, und zweite Photonen, die eine Wellenlänge innerhalb eines zweiten, anderen Wellenlängenbereichs aufweisen, umfassen, wobei jeder von dem ersten und dem zweiten Wellenlängenbereich ein anderer der folgenden ist:
ein Wellenlängenbereich, der mit einem Absorptionsmaximum von Wasser übereinstimmt;
ein Wellenlängenbereich, der mit einem Absorptionsmaximum von Lipiden übereinstimmt; und
ein Wellenlängenbereich, der mit einem Absorptionsmaximum von Beta-Karotin übereinstimmt.

2. Verarbeitungssystem (130) nach Anspruch 1, wobei:
das milchhaltige System (150) eine Brust eines menschlichen Subjekts ist; und
das Verarbeitungssystem dazu ausgebildet ist, das eine oder die mehreren Merkmale zu bestimmen durch:
Verarbeiten der Verteilung (125) von Photonenankunftszeiten, um ein zeitveränderliches Milchgangerweiterungssignal zu erzeugen; und
Verarbeiten des zeitveränderlichen Milchgangerweiterungssignals, um die eine oder mehreren Eigenschaften zu bestimmen.

3. Verarbeitungssystem (130) nach Anspruch 2, wobei:
die Photonen weiter dritte Photonen umfassen, die eine Wellenlänge innerhalb eines dritten Wellenlängenbereichs aufweisen, der sich von dem ersten und zweiten Wellenlängenbereich unterscheidet, wobei jeder von dem ersten Wellenlängenbereich, dem zweiten Wellenlängenbereich und dem dritten Wellenlängenbereich ein anderer der folgenden ist:
ein Wellenlängenbereich, der mit einem Absorptionsmaximum von Wasser übereinstimmt;
ein Wellenlängenbereich, der mit einem Absorptionsmaximum von Lipiden übereinstimmt; und
ein Wellenlängenbereich, der mit einem Absorptionsmaximum von Beta-Karotin übereinstimmt; und
wobei das Verarbeitungssystem dazu ausgebildet ist, das Milchgangerzeugungssignal zu erzeugen durch:
für ein anfängliches Zeitfenster:
Verarbeiten der Verteilung (125) von Photonenankunftszeiten, um für jedes Pixel einen ersten Absorptionskoeffizienten für jeden von dem ersten, zweiten und dritten Wellenlängenbereich zu bestimmen;
Verarbeiten der ersten Absorptionskoeffizienten, um für jedes Pixel einen Konzentrationswert für jedes von Wasser, Lipiden und Beta-Karotin zu bestimmen;
Verarbeiten der Konzentrationswerte für jedes Pixel, um Pixel zu identifizieren, die einem Milchgang entsprechen;
und für nachfolgende Zeitfenster:
Verarbeiten der Daten bezüglich der Verteilung von Photonenankunftszeiten in jedem Zeitfenster, um einen zweiten Absorptionskoeffizienten für einen von dem ersten, zweiten und dritten Wellenlängenbereich für mindestens ein Pixel der identifizierten Pixel zu bestimmen;
Verarbeiten jedes zweiten Absorptionskoeffizienten für jedes Zeitfenster für mindestens ein Pixel, um das Milchgangerweiterungssignal zu erzeugen.

4. Verarbeitungssystem (130) nach Anspruch 3, wobei:
die Photonen weiter vierte Photonen, die eine Wellenlänge innerhalb eines vierten Wellenlängenbereichs aufweisen, der mit einem Absorptionsmaximum von Oxyhämoglobin übereinstimmt, und fünfte Photonen, die eine Wellenlänge innerhalb eines fünften Wellenlängenbereichs aufweisen, der mit einem Absorptionsmaximum von Desoxyhämoglobin übereinstimmt, umfassen;
der zweite Absorptionskoeffizient für den Wellenlängenbereich bestimmt wird, der mit einem Absorptionsmaximum von Lipiden übereinstimmt;
und wobei das Verarbeitungssystem weiter dazu ausgebildet ist:
die Verteilung (125) von Photonenankunftszeiten zu verarbeiten, um einen Absorptionskoeffizienten von Oxyhämoglobin und Desoxyhämoglobin zu bestimmen; und
das Milchgangerweiterungssignal durch Verarbeiten des zweiten Absorptionskoeffizienten und des Absorptionskoeffizienten von Oxyhämoglobin und Desoxyhämoglobin zu erzeugen.

5. Verarbeitungssystem (130) nach Anspruch 2, wobei das Verarbeitungssystem dazu ausgebildet ist, das Milchgangerweiterungssignal zu erzeugen durch:
Verarbeiten der Verteilung (125) von Photonenankunftszeiten, um eine Veränderung der Verteilung im Laufe der Zeit für jeden von dem ersten und zweiten Wellenlängenbereich zu bestimmen; und
Verarbeiten der Veränderung der Verteilung im Laufe der Zeit für jeden von dem ersten und zweiten Wellenlängenbereich, um das Milchgangerweiterungssignal zu erzeugen.

6. Verarbeitungssystem (130) nach einem der Ansprüche 2 bis 5, wobei das Verarbeitungssystem weiter dazu ausgebildet ist, die Verteilung (125) von Photonenankunftszeiten basierend auf vorbestimmten anatomischen Informationen zu filtern.

7. Verarbeitungssystem (130) nach einem der Ansprüche 2 bis 6, wobei das Verarbeitungssystem dazu ausgebildet ist, das zeitveränderliche Milchgangerweiterungssignal zu verarbeiten durch:
Empfangen mindestens eines historischen, zeitveränderlichen Milchgangerweiterungssignals für das Subjekt; und
Verarbeiten des zeitveränderlichen Milchgangerweiterungssignals und des historischen zeitveränderlichen Milchgangsignals, um die eine oder mehreren Eigenschaften zu bestimmen.

8. Verarbeitungssystem (130) nach einem der Ansprüche 2 bis 7, wobei das Verarbeitungssystem dazu ausgebildet ist:
eine Vielzahl von Milchgangerweiterungssignalen zu erzeugen, die jeweils einer anderen Teilmenge von Pixeln entsprechen; und
die Vielzahl von Milchgangerweiterungssignalen zu verarbeiten, um die eine oder mehreren Eigenschaften zu bestimmen.

9. Verarbeitungssystem (130) nach einem der Ansprüche 2 bis 8, wobei:
der mindestens eine SPAD-Sensor (120) eine Vielzahl von SPAD-Sensoren umfasst, von denen jeder dazu ausgebildet ist, eine Verteilung (125) von Photonenankunftszeiten für Photonen zu erhalten, die von einem anderen Bereich der Brust reflektiert werden; und
das Verarbeitungssystem dazu ausgebildet ist:
eine Vielzahl von Milchgangerweiterungssignalen zu erzeugen, von denen jedes unter Verwendung der Verteilung von Photonenankunftszeiten eines anderen SPAD-Sensors erzeugt wird; und
die Vielzahl von Milchgangerweiterungssignalen räumlich zu korrelieren, um eine Stelle einer Milchgangverstopfung zu identifizieren.

10. Verarbeitungssystem (130) nach einem der Ansprüche 1 bis 9, wobei:
die eine oder mehreren Eigenschaften einen Lipidgehalt, einen Wassergehalt und/oder einen Beta-Karotin-Gehalt der Milch in dem milchhaltigen System umfassen;
die Photonen weiter dritte Photonen umfassen, die eine Wellenlänge innerhalb eines dritten Wellenlängenbereichs aufweisen, der sich von dem ersten und zweiten Wellenlängenbereich unterscheidet, wobei jeder von dem ersten Wellenlängenbereich, dem zweiten Wellenlängenbereich und dem dritten Wellenlängenbereich ein anderer der folgenden ist:
ein Wellenlängenbereich, der mit einem Absorptionsmaximum von Wasser übereinstimmt;
ein Wellenlängenbereich, der mit einem Absorptionsmaximum von Lipiden übereinstimmt; und
ein Wellenlängenbereich, der mit einem Absorptionsmaximum von Beta-Karotin übereinstimmt; und
das Verarbeitungssystem dazu ausgebildet ist, den Lipidgehalt, den Wassergehalt und/oder den Beta-Karotin-Gehalt der Milch im milchhaltigen System zu bestimmen durch:
Verarbeiten der Verteilung (125) von Photonenankunftszeiten, um einen Absorptionskoeffizienten für jeden von dem ersten, zweiten und dritten Wellenlängenbereich zu bestimmen;
Verarbeiten der Absorptionskoeffizienten, um einen Konzentrationswert für jedes von Wasser, Lipiden und Beta-Karotin zu bestimmen; und
Verarbeiten der Konzentrationswerte, um einen Lipidgehalt, einen Wassergehalt und/oder einen Beta-Karotin-Gehalt der Milch in dem milchhaltigen System zu bestimmen.

11. Verarbeitungssystem (130) nach einem der Ansprüche 1 bis 10, wobei das Verarbeitungssystem weiter dazu ausgebildet ist, die eine oder mehreren Eigenschaften zu verarbeiten, um eine Stillberatung zu erzeugen.

12. System (100, 600, 700, 800) zur Bestimmung einer oder mehrerer Eigenschaften eines milchhaltigen Systems (150), das menschliche Muttermilch enthält, wobei das System umfasst:
eine Lichtquelle (110), die dazu ausgebildet ist, Photonen in Richtung des milchhaltigen Systems zu emittieren, wobei die Lichtquelle dazu ausgebildet ist, Photonen zu emittieren, die erste Photonen, die eine Wellenlänge innerhalb eines ersten Wellenlängenbereichs aufweisen, und zweite Photonen, die eine Wellenlänge innerhalb eines zweiten, anderen Wellenlängenbereichs aufweisen, umfassen, **dadurch gekennzeichnet, dass** jeder von dem ersten Wellenlängenbereich und dem zweiten Wellenlängenbereich ein anderer der folgenden ist:
ein Wellenlängenbereich, der mit einem Absorptionsmaximum von Wasser übereinstimmt;
ein Wellenlängenbereich, der mit einem Absorptionsmaximum von Lipiden übereinstimmt; und
ein Wellenlängenbereich, der mit einem Absorptionsmaximum von Beta-Karotin übereinstimmt;
mindestens einen SPAD-Sensor (120), der dazu ausgebildet ist, eine Ankunftszeit von Photonen zu messen, die von innerhalb des milchhaltigen Systems reflektiert werden; und
das Verarbeitungssystem (130) nach einem der Ansprüche 1 bis 11.

13. Brustwarzen-Schnittstellenvorrichtung (160), umfassend:
einen Hohlraum, der dazu ausgebildet ist, eine Brustwarze aufzunehmen;
eine Lichtquelle (110), die dazu ausgebildet ist, Photonen in Richtung des Hohlraums zu emittieren, wobei die Lichtquelle dazu ausgebildet ist, Photonen zu emittieren, die erste Photonen, die eine Wellenlänge innerhalb eines ersten Wellenlängenbereichs aufweisen, und zweite Photonen, die eine Wellenlänge innerhalb eines zweiten, anderen Wellenlängenbereichs aufweisen, umfassen, wobei jeder von dem ersten Wellenlängenbereich und dem zweiten Wellenlängenbereich ein anderer der folgenden ist:
ein Wellenlängenbereich, der mit einem Absorptionsmaximum von Wasser übereinstimmt;
ein Wellenlängenbereich, der mit einem Absorptionsmaximum von Lipiden übereinstimmt; und
ein Wellenlängenbereich, der mit einem Absorptionsmaximum von Beta-Karotin übereinstimmt; und
mindestens einen SPAD-Sensor (120), der dazu ausgebildet ist, die Ankunftszeit von reflektierten Photonen zu messen.

14. Verfahren (500) zum Bestimmen einer oder mehrerer Eigenschaften eines milchhaltigen Systems (150), das menschliche Muttermilch enthält, **dadurch gekennzeichnet, dass** das Verfahren umfasst:
Erhalten, von mindestens einem SPAD-Sensor (120), einer Verteilung (125) von Photonenankunftszeiten an einer Vielzahl von Pixeln des mindestens einen SPAD-Sensors für Photonen, die von einer Lichtquelle (110) emittiert und von innerhalb des milchhaltigen Systems reflektiert oder durch dieses hindurch übertragen werden, wobei die Photonen erste Photonen, die eine Wellenlänge innerhalb eines ersten Wellenlängenbereichs aufweisen, und zweite Photonen, die eine Wellenlänge innerhalb eines zweiten, anderen Wellenlängenbereichs aufweisen, umfassen, wobei jeder von dem ersten Wellenlängenbereich und dem zweiten Wellenlängenbereich ein anderer der folgenden ist:
ein Wellenlängenbereich, der mit einem Absorptionsmaximum von Wasser übereinstimmt;
ein Wellenlängenbereich, der mit einem Absorptionsmaximum von Lipiden übereinstimmt; und
ein Wellenlängenbereich, der mit einem Absorptionsmaximum von Beta-Karotin übereinstimmt; und
Verarbeiten der Verteilung von Photonenankunftszeiten, um eine oder mehrere Eigenschaften des milchhaltigen Systems zu bestimmen.

15. Computerprogrammprodukt, das Computerprogrammcodemittel umfasst, die, wenn sie auf einem Verarbeitungssystem (130) nach einem der Ansprüche 1 bis 11 ausgeführt werden, das Verarbeitungssystem (130) veranlassen, alle Schritte des Verfahrens (500) nach Anspruch 14 durchzuführen.

## Revendications

1. Système de traitement (130) pour la détermination d'une ou plusieurs caractéristiques d'un système contenant du lait (150) contenant du lait maternel humain, le système de traitement étant configuré pour :
traiter une distribution de temps d'arrivée de photons pour déterminer une ou plusieurs caractéristiques du système contenant du lait,
**caractérisé en ce que** le système de traitement est configuré pour :
obtenir, à partir d'au moins un capteur SPAD (120), la distribution (125) de temps d'arrivée de photons sur une pluralité de pixels de l'au moins un capteur SPAD pour des photons émis par une source de lumière (110) et réfléchis à l'intérieur ou transmis à travers le système contenant du lait, dans lequel
les photons comprennent des premiers photons présentant une longueur d'onde comprise dans une première plage de longueurs d'onde et des deuxièmes photons présentant une longueur d'onde comprise dans une deuxième plage de longueurs d'onde différente, dans lequel chacune parmi la première plage de longueurs d'onde et la deuxième plage de longueurs d'onde est l'une différente des suivantes :
une plage de longueurs d'onde coïncidant avec un pic d'absorption de l'eau ;
une plage de longueurs d'onde coïncidant avec un pic d'absorption des lipides ; et
une plage de longueurs d'onde coïncidant avec un pic d'absorption du bêta-carotène.

2. Système de traitement (130) selon la revendication 1, dans lequel :
le système contenant du lait (150) est un sein d'un sujet humain ; et
le système de traitement est configuré pour déterminer les une ou plusieurs caractéristiques par :
le traitement de la distribution (125) de temps d'arrivée de photons pour générer un signal de dilatation de canal galactophore variable dans le temps ; et
le traitement du signal de dilatation de canal galactophore variable dans le temps pour déterminer les une ou plusieurs caractéristiques.

3. Système de traitement (130) selon la revendication 2, dans lequel :
les photons comprennent en outre des troisièmes photons présentant une longueur d'onde dans une troisième plage de longueurs d'onde, différente des première et deuxième plages de longueurs d'onde, dans lequel chacune parmi la première plage de longueurs d'onde, la deuxième plage de longueurs d'onde et la troisième plage de longueurs d'onde sont l'une différente des suivantes :
une plage de longueurs d'onde coïncidant avec un pic d'absorption de l'eau ;
une plage de longueurs d'onde coïncidant avec un pic d'absorption des lipides ; et
une plage de longueurs d'onde coïncidant avec un pic d'absorption du bêta-carotène ; et
le système de traitement est configuré pour générer le signal de génération de canal galactophore par :
pour une fenêtre temporelle initiale :
le traitement de la distribution (125) de temps d'arrivée de photons pour déterminer, pour chaque pixel, un premier coefficient d'absorption pour chacune des première, deuxième et troisième plages de longueurs d'onde ;
le traitement des premiers coefficients d'absorption pour déterminer, pour chaque pixel, une valeur de concentration pour chacun parmi l'eau, les lipides et le bêta-carotène ;
le traitement des valeurs de concentration pour chaque pixel afin d'identifier les pixels correspondant à un canal galactophore ;
et, pour des fenêtres temporelles suivantes :
le traitement des données relatives à la distribution de temps d'arrivée de photons dans chaque fenêtre temporelle afin de déterminer un deuxième coefficient d'absorption pour l'une des première, deuxième et troisième plages de longueurs d'onde pour au moins un pixel parmi les pixels identifiés ;
le traitement de chaque deuxième coefficient d'absorption pour chaque fenêtre temporelle pour au moins un pixel afin de générer le signal de dilatation de canal galactophore.

4. Système de traitement (130) selon la revendication 3, dans lequel :
les photons comprennent en outre des quatrièmes photons présentant une longueur d'onde dans une quatrième plage de longueurs d'onde coïncidant avec un pic d'absorption de l'oxyhémoglobine et des cinquièmes photons présentant une longueur d'onde dans une cinquième plage de longueurs d'onde coïncidant avec un pic d'absorption de la désoxyhémoglobine ;
le deuxième coefficient d'absorption est déterminé pour la plage de longueurs d'onde coïncidant avec un pic d'absorption des lipides ;
et le système de traitement est en outre configuré pour :
traiter la distribution (125) de temps d'arrivée de photons pour déterminer un coefficient d'absorption de l'oxyhémoglobine et de la désoxyhémoglobine ; et
générer le signal de dilatation de canal galactophore en traitant le deuxième coefficient d'absorption et le coefficient d'absorption de l'oxyhémoglobine et de la désoxyhémoglobine.

5. Système de traitement (130) selon la revendication 2, dans lequel le système de traitement est configuré pour générer le signal de dilatation de canal galactophore par :
le traitement de la distribution (125) des temps d'arrivée des photons pour déterminer une variation de la distribution au fil du temps pour chacune des première et deuxième plages de longueurs d'onde ; et
le traitement de la variation de la distribution au fil du temps pour chacune des première et deuxième plages de longueurs d'onde afin de générer le signal de dilatation de canal galactophore.

6. Système de traitement (130) selon l'une quelconque des revendications 2 à 5, dans lequel le système de traitement est en outre configuré pour filtrer la distribution (125) de temps d'arrivée de photons en fonction d'informations anatomiques prédéterminées.

7. Système de traitement (130) selon l'une quelconque des revendications 2 à 6, dans lequel le système de traitement est configuré pour traiter le signal de dilatation de canal galactophore variable dans le temps par :
la réception d'au moins un signal historique de dilatation de canal galactophore variable dans le temps pour le sujet ; et
le traitement du signal de dilatation de canal galactophore variable dans le temps et du signal historique du canal galactophore variable dans le temps pour déterminer les une ou plusieurs caractéristiques.

8. Système de traitement (130) selon l'une quelconque des revendications 2 à 7, dans lequel le système de traitement est configuré pour :
générer une pluralité de signaux de dilatation de canal galactophore, chacun correspondant à un sous-ensemble différent de pixels ; et
traiter la pluralité des signaux de dilatation de canal galactophore pour déterminer les une ou plusieurs caractéristiques.

9. Système de traitement (130) selon l'une quelconque des revendications 2 à 8, dans lequel :
l'au moins un capteur SPAD (120) comprend une pluralité de capteurs SPAD, chacun configuré pour obtenir une distribution (125) de temps d'arrivée de photons pour les photons réfléchis par une région différente du sein ; et
le système de traitement est configuré pour :
générer une pluralité de signaux de dilatation de canal galactophore, chacun généré en utilisant la distribution de temps d'arrivée de photons d'un capteur SPAD différent ; et
corréler spatialement la pluralité de signaux de dilatation de canal galactophore pour identifier un emplacement d'une obstruction de canal galactophores.

10. Système de traitement (130) selon l'une quelconque des revendications 1 à 9, dans lequel :
les une ou plusieurs caractéristiques comprennent une teneur en lipides, une teneur en eau et/ou une teneur en bêta-carotène du lait dans le système contenant du lait ;
les photons comprennent en outre des troisièmes photons présentant une longueur d'onde dans une troisième plage de longueurs d'onde, différente des première et deuxième plages de longueurs d'onde, dans lequel chacune parmi la première plage de longueurs d'onde, la deuxième plage de longueurs d'onde et la troisième plage de longueurs d'onde sont l'une différente des suivantes :
une plage de longueurs d'onde coïncidant avec un pic d'absorption de l'eau ;
une plage de longueurs d'onde coïncidant avec un pic d'absorption des lipides ; et
une plage de longueurs d'onde coïncidant avec un pic d'absorption du bêta-carotène ; et
le système de traitement est configuré pour déterminer la teneur en lipides, la teneur en eau et/ou la teneur en bêta-carotène du lait dans le système contenant du lait par :
le traitement de la distribution (125) de temps d'arrivée de photons pour déterminer un coefficient d'absorption pour chacune des première, deuxième et troisième plages de longueurs d'onde ;
le traitement des coefficients d'absorption pour déterminer une valeur de concentration pour chacun parmi l'eau, les lipides et le bêta-carotène ; et
le traitement des valeurs de concentration pour déterminer la teneur en lipides, la teneur en eau et/ou la teneur en bêta-carotène du lait dans le système contenant du lait.

11. Système de traitement (130) selon l'une quelconque des revendications 1 à 10, dans lequel le système de traitement est en outre configuré pour traiter les une ou plusieurs caractéristiques afin de générer des conseils en matière d'allaitement.

12. Système (100, 600, 700, 800) pour déterminer une ou plusieurs caractéristiques d'un système contenant du lait (150) contenant du lait maternel humain, le système comprenant :
une source de lumière (110) configurée pour émettre des photons vers le système contenant du lait, dans lequel la source de lumière est configurée pour émettre des photons comprenant des premiers photons présentant une longueur d'onde dans une première plage de longueurs d'onde et des deuxièmes photons présentant une longueur d'onde dans une deuxième plage de longueurs d'onde différente, **caractérisé en ce que** chacune parmi la première plage de longueurs d'onde et la deuxième plage de longueurs d'onde est l'une différente des suivantes :
une plage de longueurs d'onde coïncidant avec un pic d'absorption de l'eau ;
une plage de longueurs d'onde coïncidant avec un pic d'absorption des lipides ; et
une plage de longueurs d'onde coïncidant avec un pic d'absorption du bêta-carotène ;
au moins un capteur SPAD (120), configuré pour mesurer un temps d'arrivée de photons réfléchis à partir de l'intérieur du système contenant du lait ; et
le système de traitement (130) selon l'une quelconque des revendications 1 à 11.

13. Dispositif d'interface de mamelon (160), comprenant :
une cavité configurée pour recevoir un mamelon ;
une source de lumière (110) configurée pour émettre des photons vers la cavité, dans lequel la source de lumière est configurée pour émettre des photons comprenant des premiers photons présentant une longueur d'onde dans une première plage de longueurs d'onde et des deuxièmes photons présentant une longueur d'onde dans une deuxième plage de longueurs d'onde différente, dans lequel chacune parmi la première plage de longueurs d'onde et la deuxième plage de longueurs d'onde est l'une différente des suivantes :
une plage de longueurs d'onde coïncidant avec un pic d'absorption de l'eau ;
une plage de longueurs d'onde coïncidant avec un pic d'absorption des lipides ; et
une plage de longueurs d'onde coïncidant avec un pic d'absorption du bêta-carotène ; et
au moins un capteur SPAD (120), configuré pour mesurer un temps d'arrivée de photons réfléchis.

14. Procédé (500) de détermination d'une ou plusieurs caractéristiques d'un système contenant du lait (150) contenant du lait maternel humain, **caractérisé en ce que** le procédé comprend :
l'obtention, à partir d'au moins un capteur SPAD (120), d'une distribution (125) de temps d'arrivée de photons sur une pluralité de pixels de l'au moins un capteur SPAD pour des photons émis par une source de lumière (110) et réfléchis à partir de l'intérieur ou transmis à travers le système contenant du lait, dans lequel les photons comprennent des premiers photons présentant une longueur d'onde dans une première plage de longueurs d'onde et des deuxièmes photons présentant une longueur d'onde dans une deuxième plage de longueurs d'onde différente, dans lequel chacune parmi la première plage de longueurs d'onde et la deuxième plage de longueurs d'onde est l'une différente des suivantes :
une plage de longueurs d'onde coïncidant avec un pic d'absorption de l'eau ;
une plage de longueurs d'onde coïncidant avec un pic d'absorption des lipides ; et
une plage de longueurs d'onde coïncidant avec un pic d'absorption du bêta-carotène ; et
le traitement de la distribution de temps d'arrivée de photons pour déterminer une ou plusieurs caractéristiques du système contenant du lait.

15. Produit de programme informatique comprenant des moyens de code de programme informatique qui, lorsqu'ils sont exécutés sur un système de traitement (130) selon l'une quelconque des revendications 1 à 11, amènent le système de traitement (130) à réaliser toutes les étapes du procédé (500) selon la revendication 14.
